# EUROPEAN PATENT APPLICATION

(11) **EP 2 703 006 A1**
(43) Date of publication of application: **05.03.2014**
(21) Application number: 11864435.0
(22) Date of filing: 25.04.2011
(51) Int. Cl.: A61K 38/55, A61P 35/00

(54) **USE OF REGULATOR OF CALCINEURIN 1 FOR MANUFACTURING MEDICAMENT FOR TREATMENT OF DISEASES ASSOCIATED WITH INCREASED NF- B ACTIVITY**

(71) Applicant: Shandong Vigene Biosciences, Inc., Jinan, Shandong 250101 (CN)
(72) Inventor: LI, Wenbo, Changping District Beijing 102218 (CN)
(74) Representative: Hoffmann, Matthias
(86) International application number: PCT/CN2011/073261
(87) International publication number: WO 2012/145890

(57) **Abstract**

The present invention provides the use of a peptide comprising an amino acid sequence of SEQ ID NO:3, or the encoding nucleic acid thereof, for manufacturing medicament for treatment of diseases associated with increased NF-κB activity. The peptide can interact with IKB and influence phosphorylation of IKB in the tyrosine at position 42, thereby inhibiting the signal pathway of NF-κB.

Sequence Listing

<110>  SHADONG VIGENE BIOSCIENCES, INC.
       LI, Wenbo

<120>  Use of Regulator of Calcineurin 1 for Manufacturing Medicament
       for Treatment of Diseases Associated with Increased NF-kB Activity

<130>  NTD103EP

<160>  17

<170>  PatentIn version 3.3

<210>  1
<211>  197
<212>  PRT
<213>  Homo sapiens

<400>  1

Met Glu Glu Val Asp Leu Gln Asp Leu Pro Ser Ala Thr Ile Ala Cys
1               5                   10                  15

His Leu Asp Pro Arg Val Phe Val Asp Gly Leu Cys Arg Ala Lys Phe
            20                  25                  30

Glu Ser Leu Phe Arg Thr Tyr Asp Lys Asp Ile Thr Phe Gln Tyr Phe
        35                  40                  45

Lys Ser Phe Lys Arg Val Arg Ile Asn Phe Ser Asn Pro Phe Ser Ala
    50                  55                  60

Ala Asp Ala Arg Leu Gln Leu His Lys Thr Glu Phe Leu Gly Lys Glu
65                  70                  75                  80

Met Lys Leu Tyr Phe Ala Gln Thr Leu His Ile Gly Ser Ser His Leu
                85                  90                  95

Ala Pro Pro Asn Pro Asp Lys Gln Phe Leu Ile Ser Pro Pro Ala Ser
            100                 105                 110

Pro Pro Val Gly Trp Lys Gln Val Glu Asp Ala Thr Pro Val Ile Asn
        115                 120                 125

Tyr Asp Leu Leu Tyr Ala Ile Ser Lys Leu Gly Pro Gly Glu Lys Tyr
    130                 135                 140

Glu Leu His Ala Ala Thr Asp Thr Thr Pro Ser Val Val Val His Val
145                 150                 155                 160

Cys Glu Ser Asp Gln Glu Lys Glu Glu Glu Glu Glu Met Glu Arg Met
                165                 170                 175

Arg Arg Pro Lys Pro Lys Ile Ile Gln Thr Arg Arg Pro Glu Tyr Thr
            180                 185                 190

Pro Ile His Leu Ser
        195

<210>  2
<211>  197
<212>  PRT
<213>  Homo sapiens

<400>  2

Met His Phe Arg Asn Phe Asn Tyr Ser Phe Ser Ser Leu Ile Ala Cys
1               5                   10                  15

Val Ala Asn Ser Asp Ile Phe Ser Glu Ser Glu Thr Arg Ala Lys Phe
            20                  25                  30

Glu Ser Leu Phe Arg Thr Tyr Asp Lys Asp Ile Thr Phe Gln Tyr Phe
        35                  40                  45

Lys Ser Phe Lys Arg Val Arg Ile Asn Phe Ser Asn Pro Phe Ser Ala
    50                  55                  60

Ala Asp Ala Arg Leu Gln Leu His Lys Thr Glu Phe Leu Gly Lys Glu
65                  70                  75                  80

Met Lys Leu Tyr Phe Ala Gln Thr Leu His Ile Gly Ser Ser His Leu
                85                  90                  95

Ala Pro Pro Asn Pro Asp Lys Gln Phe Leu Ile Ser Pro Pro Ala Ser
            100                 105                 110

Pro Pro Val Gly Trp Lys Gln Val Glu Asp Ala Thr Pro Val Ile Asn
        115                 120                 125

Tyr Asp Leu Leu Tyr Ala Ile Ser Lys Leu Gly Pro Gly Glu Lys Tyr
    130                 135                 140

Glu Leu His Ala Ala Thr Asp Thr Thr Pro Ser Val Val Val His Val
145                 150                 155                 160

Cys Glu Ser Asp Gln Glu Lys Glu Glu Glu Glu Glu Met Glu Arg Met
                165                 170                 175

Arg Arg Pro Lys Pro Lys Ile Ile Gln Thr Arg Arg Pro Glu Tyr Thr
            180                 185                 190

Pro Ile His Leu Ser
        195

<210>  3
<211>  103
<212>  PRT
<213>  Homo sapiens

<400>  3

Met Glu Glu Val Asp Leu Gln Asp Leu Pro Ser Ala Thr Ile Ala Cys
1               5                   10                  15

His Leu Asp Pro Arg Val Phe Val Asp Gly Leu Cys Arg Ala Lys Phe
            20                  25                  30

Glu Ser Leu Phe Arg Thr Tyr Asp Lys Asp Ile Thr Phe Gln Tyr Phe
        35                  40                  45

Lys Ser Phe Lys Arg Val Arg Ile Asn Phe Ser Asn Pro Phe Ser Ala
    50                  55                  60

Ala Asp Ala Arg Leu Gln Leu His Lys Thr Glu Phe Leu Gly Lys Glu
65                  70                  75                  80

Met Lys Leu Tyr Phe Ala Gln Thr Leu His Ile Gly Ser Ser His Leu
                85                  90                  95

Ala Pro Pro Asn Pro Asp Lys
            100

<210>  4
<211>  35
<212>  DNA
<213>  Artificial

<220>
<223>  primer

<400>  4
ccgctcgagg ccaccatgga ggaggtggac ctgca                                35

<210>  5
<211>  25
<212>  DNA
<213>  Artificial

<220>
<223>  primer

<400>  5
atggtacccc tcttcttcct ccttc                                           25

<210>  6
<211>  35
<212>  DNA
<213>  Artificial

<220>
<223>  primer

<400>  6
ccgctcgagg ccaccatgca ttttagaaac tttaa                                35


<210>  7
<211>  25
<212>  DNA
<213>  Artificial

<220>
<223>  primer

<400>  7
atggtacccc tcttcttcct ccttc                                           25


<210>  8
<211>  64
<212>  DNA
<213>  Artificial

<220>
<223>  sense

<400>  8
gatccccgag gaaatggaaa gaatgaggtt caagagacct cattctttcc atttcctctt     60

ttta                                                                  64


<210>  9
<211>  64
<212>  DNA
<213>  Artificial

<220>
<223>  anti-sense

<400>  9
agcttaaaaa gaggaaatgg aaagaatgag gtctcttgaa cctcattctt tccatttcct     60

cggg                                                                  64


<210>  10
<211>  20
<212>  DNA
<213>  Artificial

<220>
<223>  primer

<400>  10

taatacgact cactataggg                                                    20


<210>  11
<211>  28
<212>  DNA
<213>  Artificial

<220>
<223>  primer

<400>  11
atggtaccgc ttgtctggat ttggcgga                                          28


<210>  12
<211>  36
<212>  DNA
<213>  Artificial

<220>
<223>  primer

<400>  12
ccgctcgagg ccaccatgca gtttctgatc tcccct                                 36


<210>  13
<211>  18
<212>  DNA
<213>  Artificial

<220>
<223>  primer

<400>  13
tagaaggcac agtcgagg                                                     18


<210>  14
<211>  10
<212>  PRT
<213>  Artificial

<220>
<223>  peptide

<400>  14

Arg Lys Lys Arg Arg Gln Arg Arg Arg Gly
1               5                   10


<210>  15
<211>  38
<212>  DNA
<213>  Artificial

<220>
<223>  oligo

<400>  15
tatgaggaag aagcggagac agcgacgaag aggatccc                               38

<210>  16
<211>  26
<212>  DNA
<213>  Artificial

<220>
<223>  primer

<400>  16
cgggatccat ggaggaggtg gacctg                                            26


<210>  17
<211>  28
<212>  DNA
<213>  Artificial

<220>
<223>  primer

<400>  17
ccgctcgagc ttgtctggat ttggcgga                                          28

## Description

### Field of the Invention

The present invention relates to the field of the treatment for human tumors and inflammations, and in particular, it relates to the inhibition of nuclear factor-kappaB (NF-κB) by regulator of calcineurin 1 (RCAN1) protein and use thereof in the treatment of diseases associated to NF-κB such as Tumors.

### Background of the Invention

In developed countries, cancer is the primary reason for death. Latest data indicate that, one out of four to five deaths is due to cancer in China. Most of the cancers do not have an efficient therapy, and cancers thus bring about serious social and economic problems world wide.

In normal physiological conditions, nuclear factor-kappaB (NF-κB) mainly participates immune responses, cell death, and inflammation responses as a transcriptional factor (Baud and Karin, 2009). NF-κB is the main cellular anti-apoptotic factor *in vivo.* Abnormities in NF-κB signaling pathway are associated to the genesis, development, and tolerance to chemical therapy and physical therapy of many tumors including hematological and solid tumors. Hematological tumors that have been discovered to have abnormally consistently increasing NF-κB activities include: multiple myeloma, acute lymphocytic leukemia, acute myelocytic leukemia, chronic lymphocytic leukemia, chronic myelocytic leukemia, mantle cell lymphoma, mucosa-associated lymphoid tissue lymphoma, diffuse large cell lymphoma, Hodgkin's lymphoma, myelodysplastic syndrome, adult T-cell leukemia. Solid tumors that have been discovered to have abnormally consistently increasing NF-κB activities include: breast cancer, ovarial cancer, lung cancer, cervical cancer, prostate cancer, liver cancer, pancreatic cancer, esophageal cancer, stomach cancer, throat cancer, bile duct cancer, thyroid cancer, parathyroid cancer, and squamous cell carcinoma in skin, head and neck (Basseres and Baldwin, 2006). Studies for mouse model of human acute T-cell leukemia have indicated that deficiencies of NF-κB can delay the genesis of leukemia (dos Santos et al., 2008). Studies have shown that many steroid hormones, naturally occurred or synthesized chemicals exert their tumor-inhibitory effects by inhibiting NF-κB.

NF-κB facilitates the genesis and development of tumors mainly by cell proliferation, angiogenesis, tumor metastasis, inflammation response, and apoptosis inhibition. Recent studies have shown that about 1/5 of multiple myeloma patients have gene mutations in NF-κB signaling pathway. In 2008, FDA has approved Bortezomib for the treatment of multiple myeloma. As a proteasome inhibitor, Bortezomib is believed to exert the anti-tumor effects mainly by inhibiting the NF-κB signaling pathway. But as a broad-spectrum proteasome inhibitor, Bortezomib has prevalent and serious side effects, which significantly limits the broad applications thereof in tumor therapies (Delforge et al., 2010). The main effect of arsenic trioxide (also referred to as white arsenic) used for acute promyelocytic leukemia is also to inhibit the activities of NF-κB, but the toxicity of arsenic trioxide also limits the application thereof in tumor therapies.

NF-κB not only plays an important role in tumors, it is also a regulatory factor for very important inflammatory factors, e.g. interleukin, tumor necrosis factor etc. Hence NF-κB also pays an important role in inflammation responses. Furthermore, as an important molecule that affects the viability of a cell, NF-κB also plays an important role in neurodegenerations including senile dementia and cerebral stroke.

Accordingly, NF-κB signaling pathway is a very important target for tumor drugs, and is also a focus for drug development world wide. Currently there exists urgent demand for drugs that can specifically inhibit the activities of NF-κB but with less side effects, which can be used in tumor therapies.

Regulator of calcineurin 1 (RCAN1) was first discovered as an endogenous inhibitory factor of calcineurin. It has been discovered that the C-terminal of RCAN1 has the activity of inhibiting calcineurin (Arron et al., 2006; Chan et al., 2005; Fuentes et al., 2000). Due to the differences in splicing, RCAN1 has 4 splicing isoforms, wherein splicing isoform 1, i.e. RCAN1.1 (SEQ ID NO:1), and splicing isoform 4, i.e. RCAN1.4 (SEQ ID NO:2) are expressed *in vivo* in relatively high level. RCAN1.1 and RCAN1.4 only have a difference of 28 amino acids in N-terminal. RCAN1.1 is relatively highly expressed in nerve tissue, while RCAN1.4 is relatively highly expressed in peripheral tissues (e.g. muscle tissue). Although these two are differently expressed in various tissues, they have similar functions.

### Summary of the Invention

In one aspect, the present invention provides use of a peptide, or an encoding nucleic acid thereof, in the preparation of a medicament for the treatment of a disease associated with increased activity of NF-κB, wherein said peptide comprises (a) the amino acid sequence of SEQ ID NO:3; or comprises (b) an amino acid sequence obtained through modification, such as substitution, deletion, or addition of one or more amino acids, in the amino acid sequence of SEQ ID NO:3, wherein said peptide has the effect of inhibiting NF-κB signalling pathway.

In one aspect, the present invention provides a pharmaceutical composition, comprsing a peptide, or an encoding nucleic acid thereof, and optionally a pharmaceutical acceptable carrier or excipient, wherein said peptide comprises (a) the amino acid sequence of SEQ ID NO:3; or comprises (b) an amino acid sequence obtained through modification, such as substitution, deletion, or addition of one or more amino acids, in the amino acid sequence of SEQ ID NO:3, wherein said peptide has the effect of inhibiting NF-κB signalling pathway.

In one aspect, the present invention provides a method for treating a disease associated with increased activity of NF-κB, comprising administrating a peptide, or an encoding nucleic acid thereof, to a patient suffering from said disease, wherein said peptide(a) comprises the amino acid sequence of SEQ ID NO:3; or (b) comprises an amino acid sequence obtained through modification, such as substitution, deletion, or addition of one or more amino acids, in the amino acid sequence of SEQ ID NO:3, wherein said peptide has the effect of inhibiting NF-κB signalling pathway.

In one embodiment, said peptide: (a) consists of the amino acid seuqence as set forth in SEQ ID NO:1, or (b) consists of the amino acid seuqence as set forth in SEQ ID NO:2, or (c) consists of the amino acid seuqence as set forth in SEQ ID NO:3, or (d)consists of an amino acid sequence obtained through modification, such as substitution, deletion, or addition of one or more amino acids, in the amino acid sequence of (a)-(c), wherein said peptide has the effect of inhibiting NF-κB signalling pathway, or (e) consists of an amino acid sequence having at least 95%, at least 96%, at least 97%, at least 98%, or at least 99% sequence identity to the amino acid sequence of (a)-(c), wherein said peptide has the effect of inhibiting NF-κB signalling pathway.

In one embodiment, said disease associated with increased activity of NF-κB is selected from: multiple myeloma, leukemia, mantle cell lymphoma, mucosa-associated lymphoid tissue lymphoma, diffuse large cell lymphoma, Hodgkin's lymphoma, myelodysplastic syndrome, breast cancer, ovarial cancer, lung cancer, cervical cancer, prostate cancer, liver cancer, pancreatic cancer, esophageal cancer, stomach cancer, throat cancer, bile duct cancer, thyroid cancer, parathyroid cancer, and squamous cell carcinoma in skin, head and neck. In one embodiment, said disease is preferably leukemia, such as leukemia selected from acute lymphocytic leukemia, acute myelocytic leukemia, chronic lymphocytic leukemia, chronic myelocytic leukemia, adult T-cell leukemia.

### Brief Description of Figures

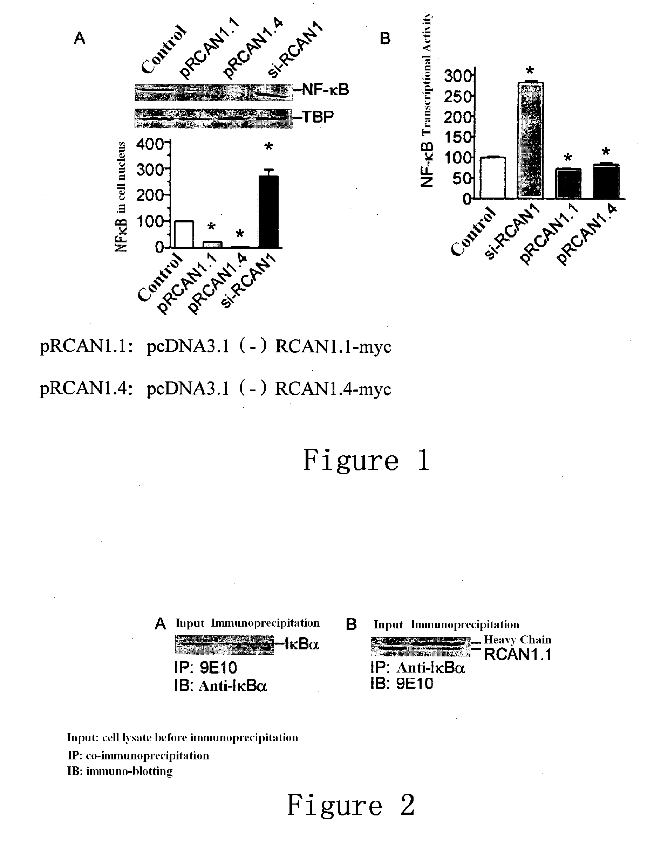

**Figure 1****: RCAN1 can decrease the nucleus transfer of NF-κB and the transcriptional activity of NF-κB.** (A) The expression vectors of RCAN1 (pcDNA3.1(-)RCAN1.1-myc and pcDNA3.1(-)RCAN1.4-myc) and the gene knock-out vector (si-RCAN1) were used to transfect HEK293 cells. The cells were collected after 48 hours, and the cell nucleus was isolated and then lysed. SDS-PAGE was conducted, and Western Bloting was conducted using anti-NF-κB antibody. TBP (TATA binding protein) detected by anti-TBP antibodywas used as the internal reference of nucleoproteins. (B) The RCAN1 expression vectors pcDNA3.1(-)RCAN1.1-myc and pcDNA3.1(-)RCAN1.4-myc, as well as the gene knock-out vectors si-RCAN1 and the indicator vector pNF-κBluc for the transcriptional activity of NF-κB were used to co-transfect HEK293. 24 hours later, luciferase kit was used to determine the luciferase activity, which was used to indicate the transcriptional activity of NF-κB in the cells. A * denotes statistical significance, p<0.05. Control: empty vector.
**Figure 2****: co-immunoprecipitation showed that there existed protein-protein interactions between RCAN1 and IKB proteins.** The RCAN1 expression vectors pcDNA3.1(-)RCAN1.1-myc was used to transfect HEK293 cells. 48 hours later, the cells were collected and lysed. (A) Anti-myc antibody (9E10) was used for immunoprecipitation, and anti-IKBα antibody was used for Western blotting; (B) Anti-IKBα antibody was used for immunoprecipitation, and anti-myc antibody (9E10) was used for Western blotting. The input was the cell lysate before immunoprecipitation.

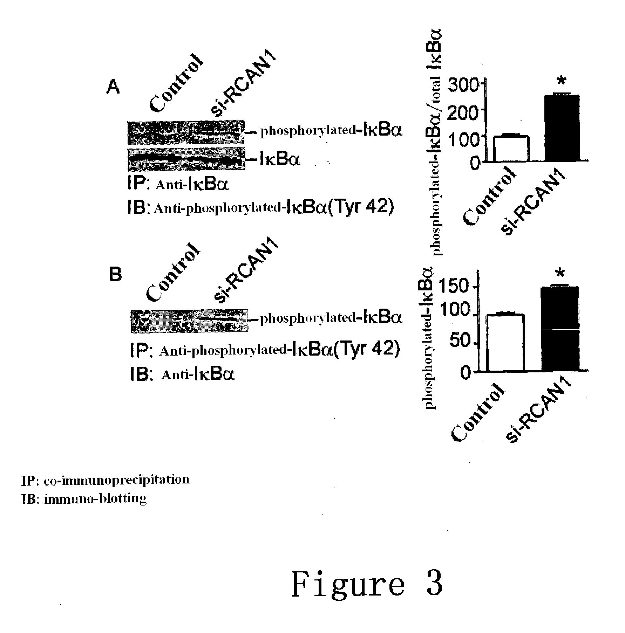

**Figure 3****: the low expression of RCAN1 allows for a significant increase in the phosphorylation of IKB at the tyrosine of position 42.** The gene knock-out vector si-RCAN1 was used to transfect HEK293 cells. 48 hours later, the cells were collected and lysed. (A) Anti-IKBα antibody was used for immunoprecipitation, and antibody of the IKB phosphorylated at the tyrosine of position 42 was used for Western blotting; (B) Antibody of the IKB phosphorylated at the tyrosine of position 42 was used for immunoprecipitation, and anti-IKBα antibody was used for Western blotting. A* denotes statistical significance, p<0.05. Control: empty vector.

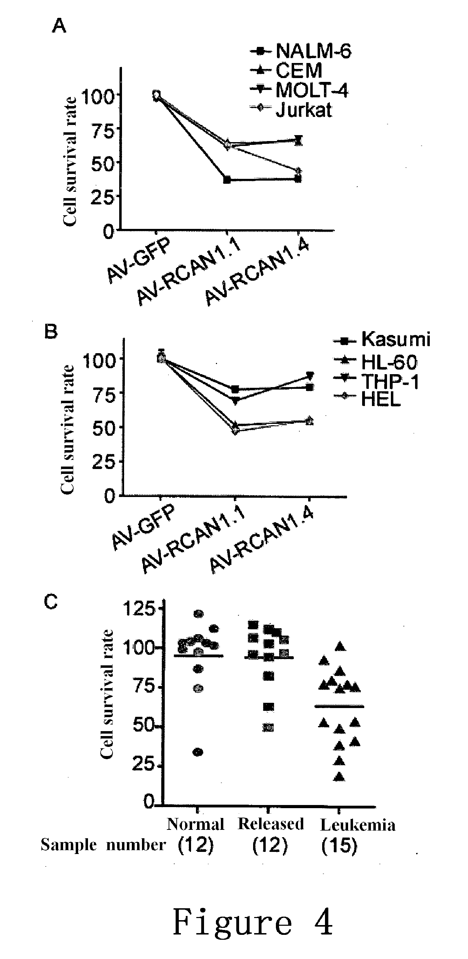

**Figure 4****: the high expression of RCAN1 resulted in death of acute leukemia cell line and primary acute leukemia cells.** The adenovirus of RCAN1 was used to transfect acute lymphocytic leukemia cell line(A) and acute myeloid lymphocytic leukemia cell line(B). 48 hours later, cell survival rate kit was used to measure the cell survival rate. (C) Mononuclear cells were isolated from the peripheral blood of leukemia patients and released patients as well as control normal population, and adenovirus of RCAN1 was used to transfect primarily cultured leukemia cells. 48 hours later, cell survival rate kit was used to measure the cell survival rate. Av-GFP was the adenovirus that expressed green fluorescent protein, Av-RCAN1.1 was the adenovirus that expressed RCAN1.1 protein (the Ad-RCAN1.1 vector as described in Example 1.2.2), and Av-RCAN1.4 was the adenovirus that expressed RCAN1.4 protein (the Ad-RCAN1.4 vector as described in Example 1.2.2).

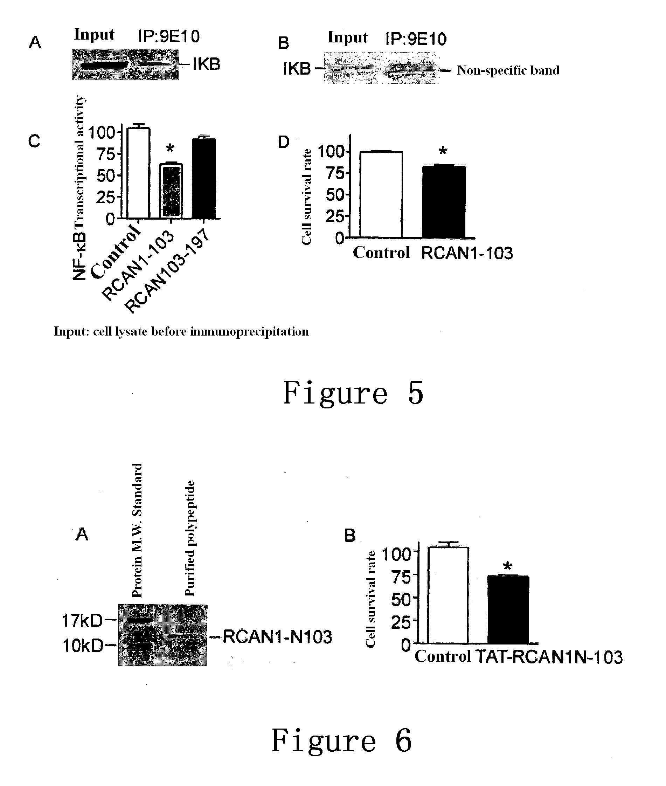

**Figure 5****: the N-terminal 103 amino acids of RCAN1 can bind to IKB, and then inhibit the transcriptional activity of NF-κB and kill acute leukemia cells.** (A) The pcDNA3.1RCAN1-103mychis vector expressing the N-terminal 103 amino acids of RCAN1, or (B) the pcDNA3.1RCAN103-197myc vector expressing amino acids 103-197 was used to transfect HEK293 cells. 48 hours later, the cells were collected and lysed. Anti-myc antibody (9E10) was used for immunoprecipitation, and anti-IKBα antibody was used for Western blotting. (C) The pcDNA3.1RCAN1-103mychis vector expressing the N-terminal 103 amino acids of RCAN1, or the pcDNA3.1RCAN103-197myc vector expressing amino acids 103-197 was used to co-transfect HEK293 cells together with the indicator vector pNF-κBluc for the transcriptional activity of NF-κB. 24 hours later, luciferase kit was used to determine the luciferase activity, which was used to indicate the transcriptional activity of NF-κB in the cells. (D) The pcDNA3.1RCAN1-103mychis vector expressing the N-terminal 103 amino acids of RCAN1, or the pcDNA3.1RCAN103-197myc plasmid expressing amino acids 103-197 was used to transfect Jurkat leukemia cell line. 48 hours later, cell survival rate kit was used to measure the cell survival rate. A * denotes statistical significance, p<0.05. Control: empty vector.
**Figure 6****: purified TAT-RCAN1-103 polypeptide can result in death of leukemia cells.** (A) SDS-PAGE indicated that the purified TAT-RCAN1-103 polypeptide was a single band. (B) TAT-RCAN1-103 polypeptide was added into Jurkat cells. 48 hours later, cell survival rate kit was used to measure the cell survival rate. A * denotes statistical significance, p<0.05. Control: empty vector.

### Detailed Description of the Invention

Unless otherwise indicated, the scientific and technical terms used herein have the meanings commonly known by a person skilled in the art. Furthermore, unless specifically required, a term in singular form should encompass the plural form thereof, and a term in plural form should encompass the singular form thereof. The above mentioned techniques and methods are typically carried out according to the conventional processes known in the art or described in the references cited in the present description, see e.g. Sambrook et al. Molecular Cloning: A Laboratory Manual (3rd ed., Cold Spring Harbor Laboratory Press, Cold Spring Harbor, N. Y. (2001)), which is incorporated herein as reference.

Without limiting by theory, the NF-κB signaling pathway *in vivo* mainly relies on the κB inhibitor IKB. The phosphorylations at positions 32 and 36 of IKB facilitate the degradation of IKB in proteasome. After degradation, IKB exposed the nuclear localization sequence of NF-κB molecule, and NF-κB molecule is then transferred into cell nucleus, so as to bins to the κB reactive element on the target gene thereof to regulate the transcription of the gene (Alkalay et al., 1995). Furthermore, studies have proved that the phosphorylation of IKB at the tyrosine of position 42 can also affect the stability of IKB, so as to affect the activities of NF-κB signaling pathway (Imbert et al., 1996; Sethi et al., 2007; Waris et al., 2003).

In the present invention, it is discovered that the splicing isoforms of RCAN1 protein, RCAN1.1 (SEQ ID NO:1) and RCAN1.4 (SEQ ID NO:2), can inhibit NF-κB signaling pathway, so as to decrease the survival of cancer cells; Further, the inventors discover that a peptide (SEQ ID NO:3) having the N-termial 103 amino acides of RCAN1.1 can interact with IKB and inhibit the activities of NF-κB, so as to decrease the survival of cancer cells. Accordingly, the inventors disclose for the first time that, the RCAN1 protein is an endogeneous regulator protein for NF-κB, it can affect NF-κB signaling pathway, and it can interact with the NF-κB inhibitor IKB and affect the phosphorylation thereof at the tyrosine of position 42.

In one aspect, the present invention providesuse of a peptide, or an encoding nucleic acid thereof, in the preparation of a medicament for the treatment of a disease associated with increased activity of NF-κB, wherein said peptide(a)comprises the amino acid sequence of SEQ ID NO:3; or (b)comprises an amino acid sequence obtained through modification, such as substitution, deletion, or addition of one or more amino acids, in the amino acid sequence of SEQ ID NO:3, wherein said peptide has the effect of inhibiting NF-κB signalling pathway.

In one embodiment, said peptide (a) consists of the amino acid sequence as set forth in SEQ ID NO:1, or (b) consists of the amino acid sequence as set forth in SEQ ID NO:2, or (c) consists of the amino acid sequence as set forth in SEQ ID NO:3, or (d) consists of an amino acid sequence obtained through modification, such as substitution, deletion, or addition of one or more amino acids, in the amino acid sequence of (a)-(c), wherein said peptide has the effect of inhibiting NF-κB signalling pathway, or (e) consists of an amino acid sequence having at least 95%, at least 96%, at least 97%, at least 98%, or at least 99% sequence identity to the amino acid sequence of (a)-(c), wherein said peptide has the effect of inhibiting NF-κB signalling pathway.

When the term "comprise" is used in the description and claims of the invention, it does not exclude other elements or steps. For the purpose of the invention, the term "consist of" is considered as an preferred embodiment of the term "comprise".

The terms "peptide", "polypeptide" and "protein" herein can be interchangeably used, referring to a polymer of amino acid residues, which comprises two or more amino acids connected by peptide bond. The polymer can be linear, branched, or cyclic, and can comprise naturally occurred amino acids and/or amino acid analogs, and it can be interrupted by non-amino acids. Typically, in the case an amino acid polymer is long (e.g. with more than 50 amino acid residues), it is preferably referred to as a polypeptide or protein; while in the case it is 50 amino acids long or even shorter, it is preferably referred to as a "peptide".

The peptide herein can be prepared by any suitable prior art processes, such as chemical synthesis, recombinant expression etc. Regarding such issue, references can be made to, e.g. Sambrook et al. Molecular Cloning: A Laboratory Manual (3rd ed., Cold Spring Harbor Laboratory Press, Cold Spring Harbor, N. Y. (2001)), and Chapter 9 "Peptide Synthesis" by Atherton and Sheppard in "Peptide Synthesis" edited by Nicholson and published by Blackwell Scientific Publications.

The "modification" herein is preferably conserved sequence modifications known in the art, including substitution, addition, or deletion of an amino acid. An amino acid modication can be introduced by standard techniques known in the art, e.g. by site-directed mutagenesis in a nucleic acid encoding the protein, molecular cloning, oligonucleotide-directed mutagenesis, and random PCR-mediated mutagenesis. Conserved amino acid substitutions include those in which an amino acid residue is replaced by another amino acid residue with similar structure or chemical properties. Families of amino acid residues with similar side chains have been determined in the art. These families inlude: amino acids with basic side chains (e.g. lysine, arginine, histidine), amino acids with acidic side chains (e.g. aspartic acid, glutamic acid), uncharged amino acids with polar side chains (e.g. asparagine, glutamine, serine, threonine, tyrosine, cysteine, tryptophan), amino acids with non-polar side chains (e.g. glycine, alanine, valine, leucine, isoleucine, proline, phenylalanine, methionine), amino acids with branched side chain (e.g. threonine, valine, isoleucine), and amino acids with aromatic side chains (e.g. tyrosine, phenylalanine, tryptophan). It is known to a person skilled in the art that other amino acid families other than the above can also be used. Similar relatively small changes can also include the deletion or insertion of amino acid, or both. Computer programs well known in the art can be used to find instructions on which amino acid residues can be substituted, inserted, or deleted without eliminating the immunological activities thereof. Computer algorithms known to a person skilled in the art, such as Gap or Bestfit, can be used to optimize the alignment of amino acid sequences and to align or define similar or same amino acid residues.

For example, the herein above mentioned substitution of one or more amino acids can be substitution(s) of amino acid residue(s) in SEQ ID NO:1, 2, or 3 with amino acid residue(s) having similar structure or chemical properties, e.g. the substitutions between: arginine and lysine; glutamic acid and aspartic acid; serine and threonine, glutamine and asparagine; valine, leucine and isoleucine.

For example, the herein above mentioned deletion of one or more amino acids can be deletion of: the amino acid at position 1 of SEQ ID NO:1, the amino acid at position 1 of SEQ ID NO:2, or the amino acid at position 9 of SEQ ID NO:3.

For example, the herein above mentioned addition of one or more amino acids can be addition of: amino acid residue Ala at position 1 of SEQ ID NO:1, amino acid redisue Ala at position 1 of SEQ ID NO:2, or amino acid residue Ala at position 9 of SEQ ID NO:3.

The term "inhibition of NF-κB signaling pathway" used herein refers to the inhibition of the activity of NF-κB as the transcriptional factor for regulating downstream target genes thereof.

The term "identity" used herein refers to the percentage corresponding to the identical amino acids at same or similar positions of two protein chains. The homology percentage can be calculated by BLAST algorithms using BLOSUM 62 array, and said algorithms can be obtained from NCBI website (http://www.ncbi.nlm.nih.gov/). Preferably, said peptide with sequence identity is a functional homolog, i.e. it exhibits significant identity (e.g. about 50% of sequence identity at amino acid level) and it can exert the same function as the corresponding protein thereof. Functional homolog with at least about 60%, at least about 70%, at least about 80%, at least about 90%, at least about 95%, at least about 96%, at least about 97%, at least about 98%, at least about 99% or higher sequence identity is preferred functional homolog.

The terms "nucleic acid", "nucleic acid molecule", "polynucleotide" and "nucleotide sequence" herein can be interchangeably used, defining polymers with any length, such as poly-deoxyribonucleotide (DNA) (e.g. cDNA, genomic DNA, plasmid, vector, isolated DNA, and any mixtures thereof) or poly-ribonucleotide (RNA) molecule (e.g. mRNA), or mixed poly-deoxyribonucleotide and poly-deoxyribonucleotide. They comprise single-strand or double strand, linear or circular, native or synthesized polynucleotides.

As for a given nuclei acid, "encoding" or "encoded" means that is contains the information of being translated into a given protein. A nucleic acid encoding a protein can contain non-translaion sequence (e.g. intron) within the translation region of the nucleic acid, or lack such inserted non-translaion sequence (e.g. in cDNA). The information for encoding a protein is assigned by codon usage.

In one embodiment, the nuclei acid of the invention can be contained in vector such as expression vector, plasmid, virus, phagemid, phage, cosmid, or artificial chromosome. As used herein, "expression vector" refers to recombinantly or synthetically produced nuclei acid construct, which has a series of given nucleic acid elements. Said nucleic acid elements allow for the transcriptiond of a particular nuclei acid in the host cell, e.g., an expression vector can not only encode the nuclei acid sequence to be expressed, but also contain replication and control sequences that are dirived form and compatible with the expression host, as well as a selective marker that confers the transfected cell a selectable phenotype. The nuclei acid of the invention can be incorporated into plasmid, chromoome, mitochondrion DNA, plastide DNA, viruse, or nucleic acid frament. Many suitable expression vectors are known in the art and are commercially available, e.g. prokaryotic expression vectors, yeast expression vectors, mammal expression vectors, insect cell expression vectors, plant cell expression vectors. In one embodiment, the nucleic acid sequenceof the invention (SEQ ID NO:1, 2, or 3) is expressed in expression vectors, such as pcDNA3.1(-)mychis(c) (Invitrogen), pSuper (Oligoengine), or Adeno-X (Clontech).

A "host cell" refers to a cell that contains a vector and supports the replication and/or expression of said vector. A host cell can be prokaryotic cell such as *E. Coli* cell, or eukaryotic cell such as yeast cell, insect cell, amphibian animal cell, or mammal cell. A host cell that expresses the protein of the invention can be any prokaryotic cells or eukaryotic cells used in the art, e.g. HEK293, HEK293T, *E. Coli* cells etc. A person skilled in the art knows various techniqus for cell culture and expression, collection, purification, and for detection of the expressed protein. With this respect, references can be made to J. Perbal, A Practical Guide to Molecular Cloning, John Wiley and Sons (1984), J. Sambrook et al., Molecular Cloning: A Laboratory Manual, Cold Spring Harbour Laboratory Press (1989) and F.M. Ausubel et al. (editors), Current Protocols in Molecular Biology, Greene Pub. Associates and Wiley-Interscience (1988), Ed Harlow and David Lane (editors).

In another aspect, the present invention also provides use of a peptide, or an encoding nucleic acid thereof, as medicament for treating a disease associated with increased activity of NF-κB, wherein said peptide(a)comprises the amino acid sequence of SEQ ID NO:3; or (b)comprises an amino acid sequence obtained through modification, such as substitution, deletion, or addition of one or more amino acids, in the amino acid sequence of SEQ ID NO:3, wherein said peptide has the effect of inhibiting NF-κB signalling pathway.

In another aspect, the present invention also provides a pharmaceutical composition for treating a disease associated with increased activity of NF-κB, which comprises a peptide, or an encoding nucleic acid thereof, and optionally a pharmaceutical acceptable carrier or excipient, wherein said peptide (a) comprises the amino acid sequence of SEQ ID NO:3; or (b) comprises an amino acid sequence obtained through modification, such as substitution, deletion, or addition of one or more amino acids, in the amino acid sequence of SEQ ID NO:3, wherein said peptide has the effect of inhibiting NF-κB signalling pathway.

In one embodiment, said peptide (a) consists of the amino acid sequence as set forth in SEQ ID NO:1, or (b) consists of the amino acid sequence as set forth in SEQ ID NO:2, or (c) consists of the amino acid sequence as set forth in SEQ ID NO:3, or (d) consists of an amino acid sequence obtained through modification, such as substitution, deletion, or addition of one or more amino acids, in the amino acid sequence of (a)-(c), wherein said peptide has the effect of inhibiting NF-κB signalling pathway, or (e) consists of an amino acid sequence having at least 95%, at least 96%, at least 97%, at least 98%, or at least 99% sequence identity to the amino acid sequence of (a)-(c), wherein said peptide has the effect of inhibiting NF-κB signalling pathway. The furmulation of a peptide or a nucleic acid into a pharmaceutical composition can be seen in Gennaro, A.L. and Gennaro, A.R. (2000) Remington: The Science and Practice of Pharmacy, 20th Ed., Lippincott Williams & Wilkins, Philadelphia, PA; Crowder, T.M. et al. (2003) A Guide to Pharmaceutical Particulate Science. Interpharm/CRC, Boca Raton, FL; and Niazi, S.K. (2004) Handbook of Pharmaceutical Manufacturing Formulations, CRC Press, Boca Raton, FL.

In another aspect, the present invention also provides a method for treating a disease associated with increased activity of NF-κB, comprising administrating to a patient sufferring from said disease a peptide, or an encoding nucleic acid thereof, or a pharmaceutical composition containing the same, wherein said peptide (a) comprises the amino acid sequence of SEQ ID NO:3; or (b) comprises an amino acid sequence obtained through modification, such as substitution, deletion, or addition of one or more amino acids, in the amino acid sequence of SEQ ID NO:3, wherein said peptide has the effect of inhibiting NF-κB signalling pathway.

The term "administration" as used herein refers to the introduction of a pre-determined amount of agent into a patient by an appropriated means. The isolated peptide, isolated nuclei acid molecule, expression vector, recombinant cell, pharmaceutical composition, or vaccine according to the invention can be administered by any conventional ways, as long as it can reach the desired tissue. Many administration ways can be considered, including intraperitoneal, intravenous, intramuscular, subcutaneous, transdermal, oral, local, intranasal, intrapulmonary, and intrarectal administration. But the invention is not limited to these exemplified administration ways.

In one embodiment, said peptide (a) consists of the amino acid sequence as set forth in SEQ ID NO:1, or (b) consists of the amino acid sequence as set forth in SEQ ID NO:2, or (c) consists of the amino acid sequence as set forth in SEQ ID NO:3, or (d) consists of an amino acid sequence obtained through modification, such as substitution, deletion, or addition of one or more amino acids, in the amino acid sequence of (a)-(c), wherein said peptide has the effect of inhibiting NF-κB signalling pathway, or (e)consists of an amino acid sequence having at least 95%, at least 96%, at least 97%, at least 98%, or at least 99% sequence identity to the amino acid sequence of (a)-(c), wherein said peptide has the effect of inhibiting NF-κB signalling pathway.

The peptide herein according to the invention can also be fused with other peptides, so that the physiological or pharmacetical properties of the peptide can be modified, e.g. the stability or membrane penetrability can be increased. In one embodiment, the peptide of the invention (e.g. SEQ ID NO:3) can be fused with the Tat protein (e.g. the amino acids of No. 47-57 RKKRRQRRRG(SEQ ID NO:14)) of human human immunodeficiency virus so as to facilitate the membrane penetrability of the polypeptide.

The disease associated with increased activity of NF-κB herein refers to any disease with the genesis, development, persistence, or recurrence thereof being associated with the increased activity of NF-κB (Basseres and Baldwin, 2006). For example, said disease can be selected from multiple myeloma, leukemia, mantle cell lymphoma, mucosa-associated lymphoid tissue lymphoma, diffuse large cell lymphoma, Hodgkin's lymphoma, myelodysplastic syndrome, breast cancer, ovarial cancer, lung cancer, cervical cancer, prostate cancer, liver cancer, pancreatic cancer, esophageal cancer, stomach cancer, throat cancer, bile duct cancer, thyroid cancer, parathyroid cancer, and squamous cell carcinoma in skin, head and neck. In one embodiment, said disease is preferably leukemia, such as leukemia selected from acute lymphocytic leukemia, acute myelocytic leukemia, chronic lymphocytic leukemia, chronic myelocytic leukemia, adult T-cell leukemia.

Therapy, as used herein, refers to abatement, release, elimination, interference, prevention of symptom of the disease, and/or delay of the genesis and/or development of the disease. The monitoring and/or measuring of symptom of the disease can be conducted by a clinician and by any means or processes.

Whether a peptide, or an encoding nucleic acid thereof, can inhibit the activity of NF-κB can be determined by measuring the amount of NF-κB protein in cell nucleus. Many assays for measuring NF-κB protein are known in the art, such as cell nucleus protein immunoblotting, electrophoretic mobility assay of isolated nucleus protein, and using reporter gene system to determine the activity of NF-κB protein as a transcriptional factor (Imbert et al., 1996). In one embodiment of the invention, Western Blotting is used to measure the amount of NF-κB protein in cell nucleus, so as to study the inhibitory effects of RCAN1 against the activity of NF-κB.

It is known in the art that many *in vivo* experiments (e.g. animal experiments) or *in vitro* experiments (e.g. cell assays) can be used to study whether a peptide, or an encoding nucleic acid thereof, are useful for the treatment of a disease associated with increased activity of NF-κB (Kordes et al., 2000). For example, leukemia is known to be one disease associated with increased activity of NF-κB. Leukemia is a type of hematological malignancies. Leukemia cell lines such as JURKAT etc. are good leukemia cell models, especially the short-term cultured primary leukemia cells, which can be immediately used in drug test after the direct isolation from peripheral blood of the patient, and which can indicate the effects and mechanisms of a drug in cellular assays based on cell survival rate. In the present invention, leukemia cell lines and primarily cultured leukemia cells are used as models for studying the effects of RCAN1 in treating leukemia, and said models can demonstrate that RCAN1 is a good drug candidate for the treatment of leukemia.

The RCAN1 protein of the invention, or the fragment of the N-terminal 103 amino acids thereof, has the following advantages as a novel NF-κB inhibitor: RCAN1 is an endogenous protein naturally occured in human, and thus has very low immunogenicity in human body and will not be rapidly removed by human immune system; polypeptide medicaments also have the properties of high bioactivity and high action specificity when compared to small molecule compounds, and thus polypeptide medicaments have lower side effects and significant therapeutic effects.

### Examples

The present invention is further illustrated by the following examples, while none of the examples or combination thereof should be construed as limiting the scope or embodiment of the invention. The scope of the invention is defined by the appended claims. Based on the description in combination with common knowledge in the art, one skilled in the art can clearly understand the scope as defined by the claims. Various modifications and alterations may be made to the invention by one skilled in the art without departing from the spirit and scope of the invention, and such modifications and alterations are also encompassed in the protection scope as defined in the appended claims.

### Example 1, Preparation of RCAN1 Expression Vectors

### 1.1 Materials

The enzymes and dNTPs for PCR amplification, were purchased from Takarra company;
The PCR amplification primers, were synthesized by Sangon Biotech, Shanghai;
Takara restriction enzymes (EcoR I, Kpn I, Hind III, Bgl II): were purchased from New England Biolab company;
Takara T4 ligase, was purchased from Takara Biotechnology co. ltd., Dalian;
Eukaryotic expression vector pcDNA3.1(-)mychis(c), was purchased from Invitrogen Company;
Gene knock-out vector pSuper, was purchased from Oligoengine;
Adenoviral expression vector Adeno-X, was purchased from Clontech;
*E. coli* DH5α was purchased from Invitrogen.

The other conventional chemical reagents were all domestically produced Analytical Reagents, unless otherwise indicated.

Cell line: HEK293 cell line was purchased from ATCC. HEK293 cells were cultured in DMEM medium containing 10% of fetal bovine serum, and the culture conditions were a constant temperature of 37°C and a cell incubator containing 5 % of CO₂. All the products for cell culture were purchased from Invitrogen.

### 1.2 Experiment Procedure

### 1.2.1 The construction of plasmids

(1) In order to construct RCAN1 highly-expressing vector, pcDNA3.1(-)RCAN1.1-myc recombinant plasmid was constructed. The plasmid was obtained by: PCR amplification of human cDNA library using the following primers, digestion by EcoR I and Kpn I, and then ligation into pcDNA3.1(-)mychis(c) vector digested by the same enzymes.
   The PCR primers for cloning RCAN1.1 were:
   Upstream primer: ccgCTCGAGgccaccATGGAGGAGG TGGACCTGCA(SEQ ID NO:4)
   Downstream primer: atggtacc CCTCTTCTTCCTCCTTC(SEQ ID NO:5)

   Human cDNA library ( purchased from Clontech Company) was used as the template, and the above primers were respectively used to amplify the gene fragment by normal PCR (PCR conditions: 94°C 10min; 30 cycles of 94°C, 30s, 55°C, 30s, 72°C, 30s; and 72°C 5min in the end). The PCR amplification fragment was then isolated by agarose gel electrophoresis and recovered. The upstream of said fragment carried an EcoRI restriction site, and the downstream thereof carried a Kpn I restriction site. The DNA fragment obtained from PCR was double-digested by EcoR I + Kpn I, and then ligated into pcDNA3.1(-)mychis(c) vector that was also double-digested by EcoR 1+Kpn I. The recombinant expression plasmid pcDNA3.1(-)RCAN1.1-myc was then obtained.
(2) Using similar process like (1), the eukaryotic expression vector pcDNA3.1(-)RCAN1.4-myc for RCAN1 homology isoform 4 was constructed.
   The primers used for cloning RCAN1.4 were:
   Upstream primer: ccgCTCGAGgccaccATGCATTTTA GAAACTTTAA (SEQ ID NO:6)
   Downstream primer: atggtacc CCTCTTCTTCCTCCTTC (SEQ ID NO:7)
(3) In order to obtain the gene knock-out vector for RCAN1, two chains of nucleotides were annealed (i.e. 95□, 5 min) and naturally cooled to form a double strand, which was then inserted into a pSuper vector digested by Hind III and Bgl II to obtain Si-RCAN1.
   The sequences of the two chains of nucleotides were:
   DS-511 super sense:
      GatccccGAGGAAATGGAAAGAATGAGGttcaagagaCCTCATTCTTTCCATTTCCTCttttta (SEQ ID NO:8)
   DS-511super

AS:AgcttaaaaaGAGGAAATGGAAAGAATGAGGTCTCTTGAACCTCATTCTTTCCATT
TCCTC ggg (SEQ ID NO:9)

### 1.2.2 The construction of adenoviral vectors

The pRCAN1.1-EGFP and pRCAN1.4-EGFP vectors were first constructed: pcDNA3.1(-)RCAN1.1-myc or pcDNA3.1(-)RCAN1.4-myc plasmid were digested by EcoRI and KpnI, and the DNA fragment of about 0.7kb was recovered and used as insert, which was inserted into a pEGFP-N3 (purchased from Clonetech Company) vector digested by the same enzymes, so as to obtain the pRCAN1.1-EGFP and pRCAN1.4-EGFP vectors.

Ad-RCAN1.1 and Ad-RCAN1.4 vectors were constructed: pRCAN1.1-EGFP and pRCAN1.4-EGFP vectors were digested by Nhe I and Not I, and the DNA fragment of about 1.4kb was recovered and used as insert, which was inserted into a pShuttle2 (purchased from Clonetech Company) vector digested by the same enzymes, so as to obtain the pShuttle2-RCAN1.1-EGFP and pShuttle2-RCAN1.4-EGFP. Then the pShuttle2-RCAN1.1-EGFP and pShuttle2-RCAN1.4-EGFP were digested by I-ceuI and PI-SceI, and the DNA fragment of about 1.4kb was recovered and used as insert, which was inserted into a Adeno-X adenoviral vector (purchased from Clonetech Company) digested by the same enzymes, so as to obtain the Ad-RCAN1.1 and Ad-RCAN1.4 vectors.

### 1.2.3 The production of adenovirus

Since leukemia cell lines cannot be transfected using normal transfection methods, adenovirus was used to express RCAN1 proteins. About 10⁶ of HEK293 cells were inoculated to a 60mm culture dish one day before the transfection. Liposome LF2000 (purchased from Invitrogen) and 5µg of Pac1 (purchased from NEB) digested viral vector Adeno-X or Ad-RCAN1.1 and Ad-RCAN1.4 were used for the cell transfection (the detailed procedure can be seen in the instructions of the LF2000 Transfection Kit of Invitrogen). 7 days later, the cells were collected and frozen-thawed 3 times to lyse the cells to obtain cell lysate containing the virus. 0.5ml of cell lysate was used to transfect the HEK293 cells inoculated in the 60mm culture dish. 48 hours later, the cells were lysed by freezing-thawing to obtain the virus. The virus obtained after twice of the infections of HEK293 by the same process was used for the experiment. The titer of the virus was about 4 X 10⁸ /ml. In the invention, unless otherwise indicated, the Multiplicity of Infection (MOI) used for cell lines and primary cells was 30.

### Example 2: the high expression of RCAN1 inhibited the activity of NF-κB, while the low expression thereof can increase the activity of NF-κB

### 2.1 Procedures for the nucleus transfer of NF-κB:

The transfection of the RCAN1 expression vector pcDNA3.1(-)RCAN1.1-myc into HEK293 cells: liposome LF2000(purchased from Invitrogen) was used for the transfection of HEK293 cells. 4µl of LF2000 was added into 100µl of opti-MEM (purchased from Invitrogen), which was mixed, 5 min later, with 100µl of the opti-MEM (with 2µg of RCAN1 expression vector pcDNA3.1(-)RCAN1.1-myc added). The mixture was then placed in still at room temperature for 15 min, and subsequently added into a culture dish (35mm in diameter) with cultured HEK293.

48 hours later, the cells were isolated and lysed: the cell nucleus was isolated and lysed using a Cell Nucleus Protein Extraction Kit (purchased from Millipore).

Proteins in the cell lysate were separated using 12% glycine SDS-PAGE (purchased from Biorad): the gel electrophoresis and membrane transfer device was mini-protean 3 vertical electrophoresis device from Biorad.

Western blotting procedure: anti-NF-κB antibody (purchased from Sigma) was used to detect the NF-κB level in cell nucleus (detailed procedure can be seen in the instructions from Sigma Company). TBP protein detected by anti-TBP antibody (purchased from Sigma Company) was used as internal reference. The detailed procedures for SDS-PAGE gel electrophoresis and Western blotting can be seen in Molecular Cloning: A Laboratory Manual, Cold Spring Harbour Laboratory Press (1989).

### 2.2 Results for nucleus transfer of NF-κB

NF-κB level in the nucleus of the cells that showed high expression of RCAN1 was clearly lower (see Figure 1), indicating that RCAN1 can clearly inhibit the nucleus transfer of NF-κB. The knock-out of RCAN1 can clearly increase the nucleus transfer of NF-κB (Figure 1A).

### 2.3 Procedures for the fluorescence indicating system of NF-κB activities

RCAN1 expression vectors together with pNF-κB-luc (purchased from Clontech), pRL-TK (purchased from Promega) were co-transfected into HEK293 cells (the ratio of plasmids used was 1:1:0.02, and the detailed procedure can be seen in the instructions of the LF2000 Transfection Kit of Invitrogen). 24 hours later, double- fluorescence reporting system (purchased from Promega) was used to test the fluorescence of the lysed cells.

### 2.4 Results for the fluorescence indicating system of NF-κB activities

The results indicated that, the high expression of RCAN1 can significantly inhibit the activity of NF-κB, and the knock-out of RCAN1 can clearly increase the activity of NF-κB (Figure 1B).

### Example 3: The binding of RCAN1 to IKB

### 3.1 Procedure

HEK293 cells were transfectd with RCAN1 expression vector (as described in Example 2 2.1);
The cells were harvested after 48 hours and were then lysed;
20 µl of co-immunoprecipitation reagent Protein A/G agarose (purchased from Santa Cruz) and 2 µl of anti-IKBα antibody (purchased from Cell Signaling) were added, 4□ shaken overnight.

2000g centrifugation, the supernatant was discarded, PBS was used to was the pellet twice, and then loading buffer was added;
12% glycine SDS-PAGE was used to separate the proteins, and the proteins were transferred onto PVDF membrane;
Anti-myc antibody (purchased from ABcam) was used in Western blotting to detect the RCAN1 proteins (detailed procedure can be seen in the instructions of ABcam).

Conversely, anti-myc antibody can be used to precipitate the proteins, and then IKB antibody was used for the Western blotting. The procedures for SDS-PAGE and Western blotting can be seen in Example 2 2.1.

### 3.2 Results

As shown in the figure, there existed interactions between the RCAN1 protein and the IKB protein (Figure 2).

### Example 4: RCAN1 affected the phosphorylation of IKB at the tyrosine of position

### 4.1 Experiment Procedure

HEK293 cells were transfected with RCAN1 knock-out vector si-RCAN1 (as described in Example 2 2.1, and the amount of the vector Si-RCAN1 was also 2ug);
48 hours later, the cells were collected and lysed. IKB antibody (purchased from Cell Signaling Company) was used for the immunoprecipitation (detailed procedures can be seen in the instructions of Cell Signaling Company);
In immunoprecipitation, the proteins were separated using 12% SDS-PAGE gel and were transferred onto PVDF membrane;
Anti-IKB-Y42 phosphorylation antibody (purchased from ABcam Company) was used in Western blotting for the detection. The procedures for SDS-PAGE and Western blotting can be seen in Example 2 2.1.

### 4.2 Results

The results indicated that the knock-out of RCAN1 can clearly increase the phosphorylation of IKB at Y42 (Figure 3).

### Example 5: the infection by RCAN1 adenovirus resulted in death of leukemia cells

### 5.1 Materials

Leukemia cell strains Jurkat, NALM-6, CEM, MOLT-4, Kasumi, HL-60, THP-1, HEL were all purchased from ATCC. Leukemia primary cells and cell strains were all cultured in RPMI 1640 medium containing 10% fetal bovine serum. The products for cell culture were all purchased from Invitrogen. The culture conditions were: a constant temperature of 37°C and a cell incubator containing 5 % of CO₂.

The isolation liquid Histopaque-1077 for mononuclearcells: was purchased from Sigma company.

Cell survival rate kit, Celltiter-Glo Luminescence Kit: was purchased from Promega Company.

### 5.2 Experiment Procedure

Histopaque reagent was used in the isolation of leukemia primary cells. 3ml of Histopaque-1077 reagent was added into 15ml of centrifuge tube, with the upper layer being the 3ml of fresh peripheral blood from leukemia patient. After centrifugation at 300g for 30 min, the mononuclearcells in the second layer were pipetted and were washed once using 10ml of PBS buffer, and were then inoculated into a culture dish.

Leukemia cell strains or primary leukemia cells were transfectd with adenovirus expressing RCAN1.1 and RCAN1.4 for 48 hours later, and cell survival rate kit was used to measure the cell survival rate.

### 5.3 Results

The results showed that, the high expression of RCAN1.1 or RCAN1.4 can induce the death of acute lymphatic leukemia cell lines Jurkat, NALM-6, CEM, MOLT-4, with the survival rates being about 30-60% (Figure 4A). The high expression of RCAN1.1 or RCAN1.4 can induce the death of acute myelocytic leukemia cell lines Kasumi, HL-60, THP-1, HEL, with the survival rates being about 40-80% (Figure 4B). The high expression of RCAN1.1 in acute leukemia primary cells can induce significant cell death, with the survival rates being about 55%, but it had no clear killing effect on normal blood cells or on the blood cells from released leukemia patients, indicating that RCAN1 can specifically cause the death of leukemia cells (Figure 4C).

### Example 6: The determination of the RCAN1 domain of NF-κB

### 6.1 Materials

The plasmid pcDNA3.1RCAN1-103mychis was constructed: the primer T7(TAATACGA CTCACTATAGGG(SEQ ID NO:10)) and the reverse primer DS103KpnR: AtggtaccG CTTGTCTGGATTTGGCGGA(SEQ ID NO:11) were used, and the pcDNA3.1(-)RCAN1.1-myc was used as the substrate, to amplify the fragment of 1-103, which was inserted into pcDNA3.1(-)mychis(c) by restriction sites EcoR 1 and Kpn1, so as to obtain the recombinant plasmid pcDNA3.1RCAN1-103mychis.

The plasmid pcDNA3.1RCAN103-197myc was constructed: the primer DSCR1-103F:ccgCTCGAGgccaccatg C AGTTTCTGAT CTCCCCT(SEQ ID NO:12) and the reverse primer BGH(TAGAAGGCACAGTCGAGG(SEQ ID NO:13)) were used, and the pcDNA3.1(-)RCAN1.1-myc was used as the substrate, to amplify the fragment of 103-197, which was inserted into pcDNA3.1(-)mychis(c) by restriction sites EcoR 1 and Kpn1, so as to obtain the recombinant plasmid pcDNA3.1RCAN103-197myc.

Nucleus transfection kit VCA-1003: was purchased from Lonza Company.

### 6.2 Experiment Procedure

□ Co-immunoprecipitation: HEK293 cells were respectively transfected with vectors pcDNA3.1RCAN1-103mychis and pcDNA3.1RCAN103-197myc that expressed N-terminal or C-terminal of RCAN1. The procedure as shown in Example 3 was used for the co-immunoprecipitation experiment.
□ Fluorescence indicating system of NF-κB activities: HEK293 cells were respectively transfected with vectors pcDNA3.1RCAN1-103mychis and pcDNA3.1RCAN103-197myc that expressed N-terminal or C-terminal of RCAN1. The procedure as shown in Example 2 was used for detecting the activities of NF-κB.
□ The survival rate assay for leukemia cell strain Jurkat: Jurkat cells were transfected by a nucleus transfection method (see the instructions of the Amaxa VCA-1003 Kit) with vector pcDNA3.1RCAN1-103mychis that that expressed N-terminal of RCAN1. The Celltiter-Glo Luminescence kit (Promega) was used for testing the cell survival rate.

### 6.3 Results

The results showed that the N-terminal 103 amino acids of RCAN1 can interact with IKB and inhibit the activities of NF-κB, while the C-terminal of RCAN1 had nothing to do with the activities of NF-κB (Figure 5A, B). The N-terminal 103 amino acids of RCAN1 can decrease the survival rate of leukemia cell strain Jurkat (Figure 5C).

### Example 7: The purification and activity test for the RCAN1 polypeptide that can inhibit NF-κB

### 7.1 Experiment Procedure

The construction of pTAT-RCAN1N-103 plasmid: TAT sequence was amino acids No. 47-57 (RKKRRQRRRG(SEQ ID NO:14)) of the Tat protein of human immunodeficiency Virus HIV. The fusion of such TAT sequence with the N-terminal polypeptide of RCAN1, can facilitate the cell membrane penetrability of the polypeptide. The encoding oligonucleotide containing TAT fragment: Tatg agg aagaagcggagacagcgacgaaga ggatcc c(SEQ ID NO:15) was inserted into prokaryotic expression vector pet-28b (purchased from Novagen) digested by Nde I and Xho I restriction enzymes (purchased from New England Biolabs), so as to obtain the vector pet-28bTAT. The pet-28bTAT vector was then digested by Bamh I and Xho I, and the PCR fragment of RCAN1 1-103 was inserted therein, wherein the PCR Upstream primer was: CGGGATCCATGGAGGAGGTGGACCTG (SEQ ID NO:16); and the Downstream primer was: CCGCTCGAGCTTGTCTGGATTTGGCGGA (SEQ ID NO:17). The PCR template was pcDNA3.1(-)RCAN1.1-myc expression vector.

The purification of RCAN1 N-103 polypeptide: 50ng of the expression vector pTAT-RCAN1N-103 was transformed into 100µl of BL21(DE3)pLysS cells (purchased from Promega), with the procedure: ice bath 30 min, heat shock 40 sec, ice bath 5 min. The clone expressing TAT-RCAN1N-103 was inoculated into 200ml of LB medium containing 100µg/ml of ampicillin, 37□ shaken at 225rpm for 16 hours; and was then inoculated into 1 liter of LB medium containing 100µg/ml of ampicillin, 37°C shaken overnight. After 5000g centrifugation for 10 min, the cell pellet was collected, and was washed once with PBS. The cell pellet was dissolved in 20ml of cell suspension liquid (containing 8M urea, 100mM NaCl, 20mM HEPES, PH8.0). 3 times of 15 sec ultrasonications were used to break the cells. After 16000g centrifugation at 4°C for 15 min, the supernatant was collected, and subjected to chromatography column HisTrapTM (purchased from Amersham Pharmacia). Cell suspension liquid containing 500mM imidazole was used to elute the proteins. After desalting by PD-10 desalting column (purchased from GE company), the elute was stored at -80□ in PBS containing 10% glycerol (purchased from Invitrogen). After SDS-PAGE gel electrophoresis, Coomassie brilliant blue staining was used to show the protein bands after isolation and purification (Figure 6A).

The thus purified TAT-RCAN1N-103 polypeptide was used to treat Jurkat cells: 10µM of TAT-RCAN1N-103 polypeptide was used to treat Jurkat cells. 24 hours later, MTT was used to detect the cell survival rate. The results indicated that, the Jurkat cells in TAT-RCAN1N-103 treated group had significant lower survival rate that the normal control group (Figure 6B).

### REFERENCES:

Alkalay, I., Yaron, A., Hatzubai, A., Orian, A., Ciechanover, A., and Ben-Neriah, Y. (1995). Stimulation-dependent I kappa B alpha phosphorylation marks the NF-kappa B inhibitor for degradation via the ubiquitin-proteasome pathway. Proc Natl Acad Sci U S A 92, 10599-10603.
Arron, J.R., Winslow, M.M., Polleri, A., Chang, C.P., Wu, H., Gao, X., Neilson, J.R., Chen, L., Heit, J.J., Kim, S.K., et al. (2006). NFAT dysregulation by increased dosage of DSCR1 and DYRK1A on chromosome 21. Nature 441, 595-600.
Basseres, D.S., and Baldwin, A.S. (2006). Nuclear factor-kappaB and inhibitor of kappaB kinase pathways in oncogenic initiation and progression. Oncogene 25, 6817-6830.
Baud, V., and Karin, M. (2009). Is NF-kappaB a good target for cancer therapy? Hopes and pitfalls. Nat Rev Drug Discov 8, 33-40.
Chan, B., Greenan, G., McKeon, F., and Ellenberger, T. (2005). Identification of a peptide fragment of DSCR1 that competitively inhibits calcineurin activity in vitro and in vivo. Proc Natl Acad Sci U S A 102, 13075-13080.
Delforge, M., Blade, J., Dimopoulos, M.A., Facon, T., Kropff, M., Ludwig, H., Palumbo, A., Van Damme, P., San-Miguel, J.F., and Sonneveld, P. (2010). Treatment-related peripheral neuropathy in multiple myeloma: the challenge continues. Lancet Oncol 11, 1086-1095.
dos Santos, N.R., Williame, M., Gachet, S., Cormier, F., Janin, A., Weih, D., Weih, F., and Ghysdael, J. (2008). RelB-dependent stromal cells promote T-cell leukemogenesis. PLoS One 3, e2555.
Fuentes, J.J., Genesca, L., Kingsbury, T.J., Cunningham, K.W., Perez-Riba, M., Estivill, X., and de la Luna, S. (2000). DSCR1, overexpressed in Down syndrome, is an inhibitor of calcineurin-mediated signaling pathways. Hum Mol Genet 9, 1681-1690.
Imbert, V., Rupec, R.A., Livolsi, A., Pahl, H.L., Traenckner, E.B., Mueller-Dieckmann, C., Farahifar, D., Rossi, B., Auberger, P., Baeuerle, P.A., et al. (1996). Tyrosine phosphorylation of I kappa B-alpha activates NF-kappa B without proteolytic degradation of I kappa B-alpha. Cell 86, 787-798.
Kordes, U., Krappmann, D., Heissmeyer, V., Ludwig, W.D., and Scheidereit, C. (2000). Transcription factor NF-kappaB is constitutively activated in acute lymphoblastic leukemia cells. Leukemia 14, 399-402.
Sethi, G., Ahn, K.S., Chaturvedi, M.M., and Aggarwal, B.B. (2007). Epidermal growth factor (EGF) activates nuclear factor-kappaB through IkappaBalpha kinase-independent but EGF receptor-kinase dependent tyrosine 42 phosphorylation of IkappaBalpha. Oncogene 26, 7324-7332.
Waris, G., Livolsi, A., Imbert, V., Peyron, J.F., and Siddiqui, A. (2003). Hepatitis C virus NS5A and subgenomic replicon activate NF-kappaB via tyrosine phosphorylation of IkappaBalpha and its degradation by calpain protease. J Biol Chem 278, 40778-40787.

## Claims

1. Use of a peptide, or an encoding nucleic acid thereof, in the preparation of a medicament for the treatment of a disease associated with increased activity of NF-κB, wherein said peptide
(a) comprises the amino acid sequence of SEQ ID NO:3; or
(b) comprises an amino acid sequence obtained through modification, such as substitution, deletion, or addition of one or more amino acids, in the amino acid sequence of SEQ ID NO:3, and said peptide inhibits the NF-κB signalling pathway.

2. The use of claim 1, wherein said peptide
(a) consists of the amino acid sequence as set forth in SEQ ID NO:1, or
(b) consists of the amino acid sequence as set forth in SEQ ID NO:2, or
(c) consists of the amino acid sequence as set forth in SEQ ID NO:3, or
(d) consists of an amino acid sequence obtained through modification, such as substitution, deletion, or addition of one or more amino acids, in the amino acid sequence of (a)-(c), and said peptide inhibits the NF-κB signalling pathway, or
(e) consists of an amino acid sequence having at least 95%, at least 96%, at least 97%, at least 98%, or at least 99% sequence identity to the amino acid sequence of (a)-(c), and said peptide inhibits the NF-κB signalling pathway.

3. The use of claim 1 or 2, wherein said disease associated with increased activity of NF-κB is selected from the group consisting of: multiple myeloma, leukemia, mantle cell lymphoma, mucosa-associated lymphoid tissue lymphoma, diffuse large cell lymphoma, Hodgkin's lymphoma, myelodysplastic syndrome, breast cancer, ovarial cancer, lung cancer, cervical cancer, prostate cancer, liver cancer, pancreatic cancer, esophageal cancer, stomach cancer, throat cancer, bile duct cancer, thyroid cancer, parathyroid cancer, and squamous cell carcinoma in skin, head and neck.

4. The use of claim 3, wherein said disease is leukemia, such as a leukemia selected from the group consisting of acute lymphocytic leukemia, acute myelocytic leukemia, chronic lymphocytic leukemia, chronic myelocytic leukemia, and adult T-cell leukemia.

5. A pharmaceutical composition, comprising a peptide or an encoding nucleic acid thereof, and optionally a pharmaceutical acceptable carrier or excipient, wherein said peptide
(a) comprises the amino acid sequence of SEQ ID NO:3; or
(b) comprises an amino acid sequence obtained through modification, such as substitution, deletion, or addition of one or more amino acids, in the amino acid sequence of SEQ ID NO:3, and said peptide inhibits the NF-κB signalling pathway.

6. The pharmaceutical composition of claim 5, wherein said peptide
(a) consists of the amino acid sequence as set forth in SEQ ID NO:1, or
(b) consists of the amino acid sequence as set forth in SEQ ID NO:2, or
(c) consists of the amino acid sequence as set forth in SEQ ID NO:3, or
(d) consists of an amino acid sequence obtained through modification, such as substitution, deletion, or addition of one or more amino acids, in the amino acid sequence of (a)-(c), and said peptide inhibits the NF-κB signalling pathway, or
(e) consists of an amino acid sequence having at least 95%, at least 96%, at least 97%, at least 98%, or at least 99% sequence identity to the amino acid sequence of (a)-(c), and said peptide inhibits the NF-κB signalling pathway.

7. A method for treating a disease associated with increased activity of NF-κB, comprising administrating a peptide, or an encoding nucleic acid thereof, to a patient suffering from said disease, wherein said peptide
(a) comprises the amino acid sequence of SEQ ID NO:3; or
(b) comprises an amino acid sequence obtained through modification, such as substitution, deletion, or addition of one or more amino acids, in the amino acid sequence of SEQ ID NO:3, and said peptide inhibits the NF-κB signalling pathway.

8. The method of claim 7, wherein said peptide
(a) consists of the amino acid sequence as set forth in SEQ ID NO:1, or
(b) consists of the amino acid sequence as set forth in SEQ ID NO:2, or
(c) consists of the amino acid sequence as set forth in SEQ ID NO:3, or
(d) consists of an amino acid sequence obtained through modification, such as substitution, deletion, or addition of one or more amino acids, in the amino acid sequence of (a)-(c), and said peptide inhibits the NF-κB signalling pathway, or
(e) consists of an amino acid sequence having at least 95%, at least 96%, at least 97%, at least 98%, or at least 99% sequence identity to the amino acid sequence of (a)-(c), and said peptide inhibits the NF-κB signalling pathway.

9. The method of claim 7 or 8, wherein said disease associated with increased activity of NF-κB is selected from the group consisting of: multiple myeloma, leukemia, mantle cell lymphoma, mucosa-associated lymphoid tissue lymphoma, diffuse large cell lymphoma, Hodgkin's lymphoma, myelodysplastic syndrome, breast cancer, ovarial cancer, lung cancer, cervical cancer, prostate cancer, liver cancer, pancreatic cancer, esophageal cancer, stomach cancer, throat cancer, bile duct cancer, thyroid cancer, parathyroid cance, and squamous cell carcinoma in skin, head and neck.

10. The method of claim 9, wherein said disease is leukemia, such as a leukemia selected from the group consisting of acute lymphocytic leukemia, acute myelocytic leukemia, chronic lymphocytic leukemia, chronic myelocytic leukemia, and adult T-cell leukemia.
